(19) Europäisches Patentamt
European Patent Office
Office européen des brevets

(11) Publication number: 0 447 186 A1

# (12) EUROPEAN PATENT APPLICATION

(21) Application number: 91302070.7

(51) Int. Cl.$^5$: C07K 7/56, A61K 37/02

(22) Date of filing: 12.03.91

The applicant has filed a statement in accordance with Rule 28 (4) EPC (issue of a sample only to an expert). Accession number(s) of the deposit(s): ATCC 53942; ATCC 20868; ATCC 20957.

(30) Priority: 12.03.90 US 492012
06.11.90 US 609971

(43) Date of publication of application:
18.09.91 Bulletin 91/38

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Hammond, Milton L.
50 Stony Brook Road
Somerville, NJ 08876 (US)

(74) Representative: Thompson, John Dr. et al
Merck & Co., Inc. European Patent
Department Terlings Park Eastwick Road
Harlow, Essex CM20 2QR (GB)

## (54) N-acylated cyclohexapeptide compounds.

(57) Compounds of the formula

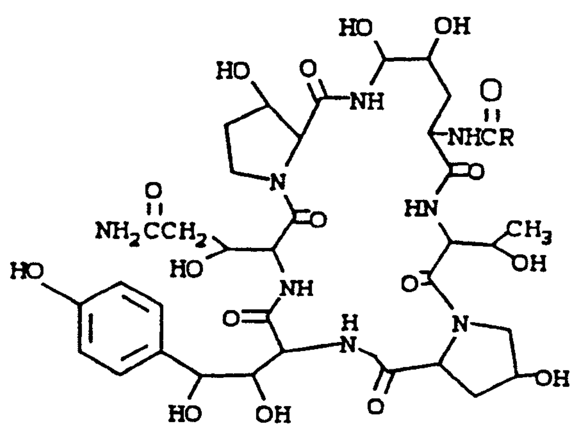

(I)

wherein R is a residue of a carboxylic acid are described. The compounds are antimicrobial agents.

EP 0 447 186 A1

Jouve, 18, rue Saint-Denis, 75001 PARIS

## N-ACYLATED CYCLOHEXAPEPTIDE COMPOUNDS

### BACKGROUND OF THE INVENTION

The present invention is directed to novel semi-synthetic compounds having the formula

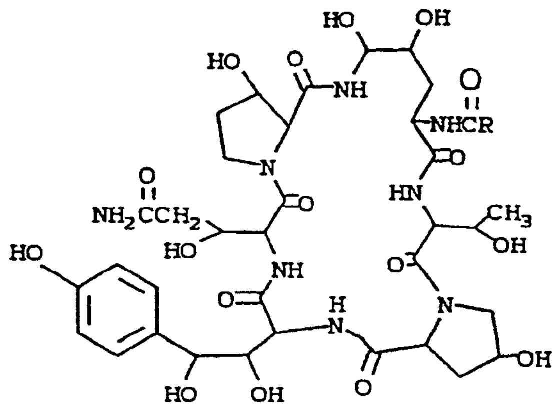

(I)

wherein R is

a) a straight or branched chain alkyl from 5 to 23 carbon atoms;

b) a straight or branched chain alkenyl from 5 to 23 carbon atoms;

c) aryl, preferably phenyl and substituted phenyl wherein the substituent is selected from $C_1$ to $C_{10}$ alkyl, $C_1$ to $C_{10}$ alkoxy or $C_1$ to $C_{10}$ thioalkoxy; and

d) heteroaryl, preferably pyrryl, thiophenyl, furyl, indolyl, benzothiophenyl, benzofuryl, imidazolyl, benzimidazolyl or pyridinyl

Representative alkyls are normal and branched octadecyl, hexadecyl, dodecyl, decyl, tetradecyl, tridecyl, pentadecyl and the like.

Representative R groups when R is alkenyl are 8,11-heptadecadienyl, 2-hexenyl, 4-octenyl, 7-pentadecenyl, 8-heptadecenyl, 10-heptadecenyl and the like.

Representative R groups when R is aryl and substituted aryl are phenyl, tolyl, xylyl, 2-ethylphenyl, 4-ethylphenyl, 4-isopropylphenyl, 4-isooctylphenyl, 4-tert-butylphenyl, 4-decylphenyl, 3-ethoxyphenyl, 4-isopropoxyphenyl, 4-(n-nonyloxy)phenyl, 4-(n-octyloxy)phenyl, 4-(n-decyloxy)phenyl, 2,4-dimethoxyphenyl, 4-(t-butoxy)phenyl, 2-methylthiophenyl, 4-(n-nonylthio)phenyl, 4-(n-octylthio)phenyl, mesityl and the like.

Representative R groups when R is heteroaryl are 2-pyrryl, 3-pyrryl, 2-furyl, 3-furyl, 2-pyridinyl, 3-pyridinyl, 4-pyridinyl, 2-indolyl, 2-benzofuryl, 2-benzimidazolyl, 2-imidazolyl, thiophene-2-yl, and the like.

The expression "Compound I" may be employed hereinafter to refer to the generic group and hence all the compounds embraced by formula (I).

The compounds are prepared by the acylation of a cyclohexapeptide compound, Compound A.

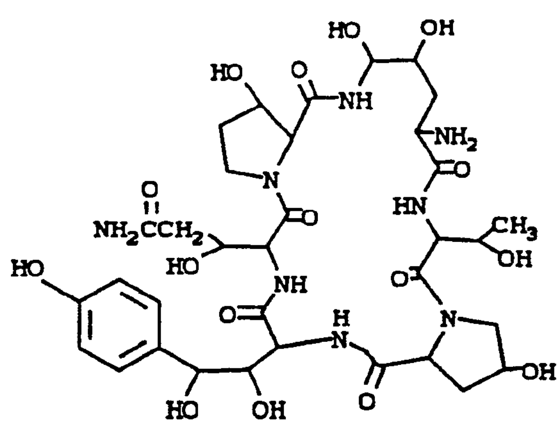

(A)

The compound of Formula A is produced by the enzymatic deacylation of antibiotic 1-[-4,5-dihydroxy-N2-

(10,12-dimethyl-1-oxotetradecyl)-L-ornithine]-5-(3-hydroxy-L-glutamine)-6-[3-hydroxy-L-proline]echinocandin B (Compound B).

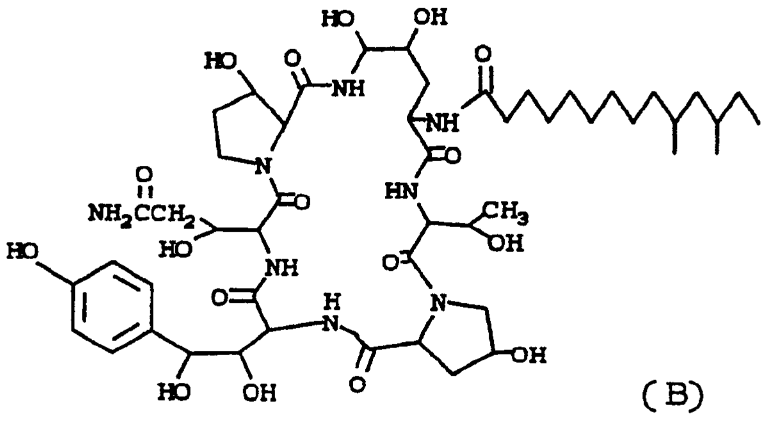

(B)

The preparation of Compound A is carried out by subjecting Compound B in a nutrient medium or a buffer solution to a deacylating enzyme obtained from or present in intact cells of a microorganism of the family Actinoplanaceae or Pseudomondacea generally at a temperature in the range of 20° to 40°C, preferably 25° to 30°C at a pH between about 5.0 and 8.0, with agitation and aeration, for from 16 to 48 hours if Pseudmondacea is used or from 40 to 60 hours if Actinoplanacea is used until the deacylation is judged to be complete as indicated by the disappearance of the anti-Candida activity of the substrate or as determined by analytical HPLC assay from a previously determined standard. The preparation of Compound A by the deacylation of Compound B using P. acidovorans ATCC 53942 is hereinafter more fully described and also is described and claimed copending application Serial No. 492,001 (Attorney Docket No. 17996) the teachings of which are incorporated by reference.

Compound B may be prepared by the cultivation of Zalerion arboricola ATCC 20868 or mutant ATCC 20957 (deposited at the American Type Culture Collection, 12301 Parklawn Drive, Rockville, MD 20852) in a nutrient medium providing sources of carbon, nitrogen and inorganic salts, preferably in a medium having a polyol, for 7 to 14 days with or without agitation, then recovering the desired metabolite by adding methanol and preferably partitioning into an oxygenated solvent such as ethyl acetate, thereafter removing the solvent and dissolving the residue in a solvent suitable for one or more chromatographic separations as hereinafter more fully described and also described in copending applications Serial No. 374,416, Serial No. 492,025, Serial No. 492,026 and Serial No. 492,024, corresponding to EP-A-0405997, the teachings of which are also incorporated by reference.

The preparation of Compound I from the cyclohexapeptide (Compound A) may be carried out by intimately contacting Compound A with an active ester

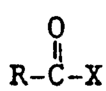

in a solvent. In the formula, R is as previously defined and X is any appropriate leaving group such as chloride, fluoride, bromide, cyanide, 1-benzotriazolate, pentachlorophenoxide, trichlorophenoxide, pivaloate, alkylsulfonate or arylsulfonate.

Suitable solvents include water, dioxane, dimethylformamide, dichloromethane, chloroform, 1,2-dichloroethane and various ethers.

In carrying out the reaction, Compound A, either as a purified compound or as a partially purified sample obtained from the deacylation of Compound B, is dissolved in dry dimethylformamide and the resulting solution intimately contacted with RCOX. The order of addition is not critical. The resulting mixture is stirred at room temperature for about 16 to 20 hours (conveniently overnight) whereupon a reaction takes place with the formation of Compound I. At the end of this period, the dimethylformamide is removed by concentrating in vacuo at 40°C and the resulting residue triturated, preferably sequentially, twice with ether, and then filtered. The filter cake product is generally a free flowing solid and is purified by reverse phase HPLC, typically in 0.5 gram aliquots employing acetonitrile/water as eluting agent. Fractions containing the purified product as determined by C. albicans assay, by U.V. or by refractive index are combined and first concentrated under reduced pressure

and then lyophilized to separate the product. Candida albicans bioassay is useful inasmuch as the non-acylated cyclohexapeptide compound is inactive against Candida albicans.

The compounds of the present invention are antimicrobial agents and useful as antimycotic agents and antiparasital agents for the control of organisms infecting mammals. They are especially useful for the control mycotic infections caused by fungal species such as C. albicans, C. parapsilosis, C. tropicalis, C. pseudotropicalis, Cryptococcus neoformans, C. guilliermondii, Saccharomyces cerevisiae Aspergillus fumigatus and the like, and for the control of Pneumocystis carinii.

The usefulness for the control of fungi causing mycotic infections may be determined, for examples, in a microbroth dilution assay employing as medium a Yeast Nitrogen Base (Difco) with 1% dextrose (YNBD). In such assay, Compound I is solubilized in 10 percent dimethyl sulfoxide (DMSO) and diluted to 2560 μg/ml/ The compounds are then diluted to 256 μg/ml in YNBD. 0.15 ml of the suspension are dispensed to the top row of a 96-well plate (each well containing 0.15 ml of YNDB) resulting in a drug concentration of 128 μg/ml.

The yeast cultures, maintained on Sabouraud dextrose agar are transferred to YM broth (Difco) and incubated overnight at 35°C with shaking (250 rpm). After incubation, each culture is diluted in sterile water to yield a final concentration of 1-5 x $10^6$ colony forming units (CFU)/ml.

96-well micro-plates are inoculated using a MIC-2000 (Dynotech) which delivers 1.5 μl per well yielding a final inoculum per well of 1.5-7.5 x $10^3$ cells. The microplates are incubated at 35°C for 24 hours. The minimum inhibitory concentrations of drug showng no visible growth.

After recording the MIC, the plates are shaken to resuspend the cells. There after, 1.5 μl samples from the wells in the 96-well microplate are transferred to a single well tray containing Sabouraud dextrose agar. The inoculated trays are incubated 24 hours at 28°C and then read. The MFC is defined as the lowest concentration of drug showing no growth or less than 4 colonies per spot.

In such assays compounds were found to have an MFC of from 0.125 to 16 μg/mL against Candida albicans.

The compounds also show activity in the inhibiting 1,3-glucan synthase which indicates anti-pneumocystis activity as well as antifungal activity. The 1,3-β-glucan synthesis assay may be carried out using protoplasts of Candida albicans in 80 μl of a mixture of 125 mM tris•HCl (pH 7.0), 0.25 mM dithiothreitol, 0.15 mM phenylmethylsulfonyl fluroide, 0.40 M glycerol, 0.75 mM EDTA, 1.0 percent bovine serum albumin, 40.0 nM guanosine 5'-[α-thio]-trisphosphate (tetralithium salt) and determining $IC_{50}$ as more fully described in Proc. Natl. Acad. Sci. USA 87 (1990), p 5950.

In such assay compounds were found to have $IC_{50}$ of from 0.3, 1.0 and 3.0 μM.

In addition to the use of the compounds as therapeutic agents in the treatment of mycotic infections in mammals they may be used wherever growth of fungi is desired to be controlled, e.g. consumer goods, plants and plant parts, plant products, wood and lumber, pulps and paper, and the like.

When employed for controlling mycotic infections, a therapeutically effective amount is administered but the actual dosage may be varied according to the particular compound employed, the physical condition of the subject being treated, and the severity and nature of the infection. Therapy is usually started at a low dosage and increased to achieve the desired effect.

The compounds may be administered parenterally, orally, topically, by inhalation, by insufflation or by other means for drug administration.

The outstanding properties are most effectively utilized when the compounds are formulated into novel pharmaceutical compositions with a pharmaceutically acceptable carrier according to conventional pharmaceutical compounding techniques.

The novel compositions contain at least a therapeutically effective amount of Compound I, usually at least 1 percent although concentrate compositions may contain up to 90 percent by weight. In preparing the compositions, Compound I is intimately admixed with any of the usual pharmaceutical media.

The compositions may be prepared in oral dosage form. For liquid preparations, Compound I is formulated with liquid carriers such as water, glycols, oils, alcohols, and the like; and for solid preparations such as capsules and tablets, Compound I is formulated with solid carriers such as starches, sugars, kaolin, ethyl cellulose, calcium and sodium carbonate, calcium phosphate, kaolin, talc, lactose, generally also with lubricant such as calcium stearate, together with binders, disintegrating agents and the like. Because of their ease in administration, tablets and capsules represent the most advantageous oral dosage form. It is especially advantageous to formulate the compositions in unit dosage form for ease of administration and uniformity of dosage.

Compound I may be formulated in compositions for injection and may be presented in unit dosage form in ampoules or in multidose containers, if necessary with an added preservative. The compositions may also take such forms as suspensions, solutions or emulsions in oily or aqueous vehicles such as 0.85 percent sodium chloride or 5 percent dextrose in water, and may contain formulating agents such as suspending, stabilizing and/or dispersing agents. Buffering agents as well as additives such as saline or glucose may be added to make

the solutions isotonic. Alternatively, the active ingredients may be in powder form for reconstituting with a suitable vehicle prior to administration.

The term "unit dosage form" refer to physically discrete units, each unit containing a predetermined quantity of active ingredient calculated to produce the desired therapeutic effect in association with the pharmaceutical carrier. Examples of such unit dosage forms are tablets, capsules, pills, powder packets, wafers, measured units in ampoules or in multidose containers and the like. A unit dosage form for antifungal application may contain from 100-200 milligrams of one of the compounds.

If the application is to be topical, the compound may be formulated in conventional creams and ointments such as white petrolatum, anhydrous lanolin, cetyl alcohol, cold cream, glyceryl monostearate, rose water and the like. Usually a 1 to 2 percent cream or solution is prepared and applied to the area to be treated.

The following examples illustrate the invention but are not to be construed as limiting:

EXAMPLE I

1-[4,5-Dihydroxy-N$^2$-(1-oxo[4-n-octyloxybenzoyl])-ornithine]-5-(3-hydroxyglutamine)-6-[3-hydroxyproline]-echinocandin B (Ia)

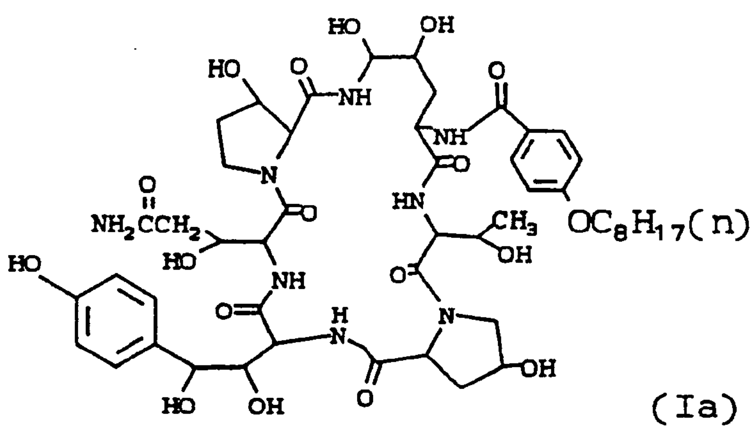

(Ia)

A. Preparation of pentafluorophenyl 4-(n-octyloxy)benzoate

To a suspension of 7.50 grams (30.0 mmol) of 4-(n-octyloxy)benzoic acid in 50 milliliters of ethyl acetate was added 6.07 grams (33.0 mmol) of pentafluorophenol. The resulting mixture was cooled in an ice bath and to the cooled solution was added with stirring, 6.46 grams (33.0 mmol) of dicyclohexylcarbodiimide. The mixture was stirred at room temperature for four hours whereupon a reaction took place with the formation of pentafluorophenyl 4-(n-octyloxy)benzoate and dicyclohexylurea. The latter, which precipitated in the reaction mixture, was removed by filtration, the filter cake washed with ethyl acetate, and the filtrate and ethyl acetate wash combined, partitioned with water and the organic solution dried over sodium sulfate. The dried solution was subjected to reduced pressure to remove the solvent and to obtain 11.80 grams (94 percent yield) of the pentafluorophenyl 4-(n-octyloxy)benzoate as a white crystalline solid, m.p. 46-47°C.

B. Preparation of Compound Ia

5.0 grams of semipurified Compound A, obtained by deacylation of Compound B, in a manner hereinafter described is dissolved in 60 milliliters of dimethylformamide (DMF). A 15 milliliter aliquot of Compound A is added to 0.948 gram of the pentafluorophenyl ester of 4-(n-octyloxy)benzoic acid, prepared as described in part A, and the mixture allowed to stir overnight (about 16 hours) at room temperature. At the end of this period, the dimethylformamide is removed by concentrating the mixture under reduced pressure at 40°C to obtain Compound Ia as residue. The residue is triturated twice with ether, twice with methylene chloride, again with ether and thereafter purified by preparative HPLC over a "Zorbax" (DuPont) C8 1" diameter column in 0.5 gram aliquots and eluting with acetonitrile/water (40/60). Eluate fractions containing active ingredient as determined by C. albicans assay are combined and concentrated, first under reduced pressure and then by lyophilization to obtain Compound Ia, molecular weight 1058, as a solid.

EXAMPLE II

1-[4,5-Dihydroxy-N2-(1-oxo-9,12-octadecadienyl)-ornithine]-5-(3-hydroxyglutamine)-6-[3-hydroxyproline]-echinocandin B (Ib)

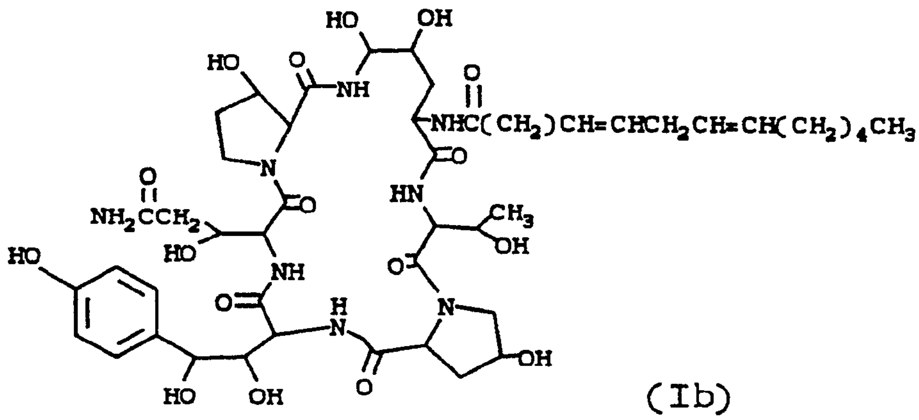

(Ib)

A. Preparation of pentafluorophenyl linoleate

In an operation carried out in a manner similar to that described in Example I, pentafluorophenyl linoleate is obtained by the reaction of 0.636 gram (2.27 mmol) of linoleic acid, 0.515 gram of dicyclohexylcarbodiimide and 0.460 gram of pentafluorophenol in 4 milliliters of ethyl acetate. The ester is a light colored solid.

B. Preparation of Compound Ib

In an operation carried out in a manner similar to that described in Example I, a 15 milliliter aliquot of the solution of semipurified Compound A in dimethylformamide prepared as in Example I, is added to the pentafluorophenyl linoleate prepared in Part A and the mixture stirred overnight at room temperature. Compound Ib is formed in the reaction mixture and is recovered by removing the dimethylformamide by vaporization under reduced pressure, working up the residue, purifying by preparative HPLC over a "Zorbax" column, and thereafter recovering by concentrating and lyophilizing the eluates to obtain a solid of m.w. 1088.

EXAMPLE III

1-[4,5-Dihydroxy-N2-(1-oxo-octadec-9-enyl)-ornithine]-5-(3-hydroxyglutamine)-6-[3-hydroxyproline]echinocandin B (Ic).

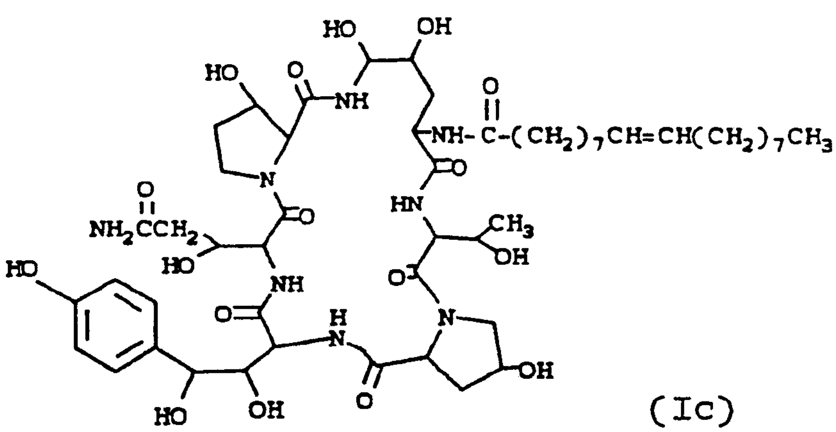

(Ic)

A. Preparation of pentafluorophenyl oleate

In an operation carried out in a manner similar to that previously described, pentafluorophenyl oleate is obtained by the reaction of 0.647 gram (2.27 mmol) of oleic acid and equivalent amounts of dicyclohexylcarbodiimide and pentafluorophenol. After working up the reaction mixture, pentafluorophenyl oleate is recovered

as a light colored residue.

B. Preparation of Compound Ic

In a manner similar to that described in Examples I and II, a 15 milliliter aliquot of the solution of semipurified Compound A in dimethylformamide prepared as described in Example I is added to pentafluorophenyl oleate prepared in Part A and the mixture stirred overnight at room temperature to form Compound Ic in the reaction mixture. The reaction mixture is subjected to reduced pressure to vaporize the dimethylformamide, and the residue triturated with ether and methylene chloride, purified by preparative HPLC and the eluates concentrated and lyophilized to obtain Compound Ic, m.w. 1090.

EXAMPLE IV

In a manner similar to that described in the foregoing examples, 1-[4,5-dihydroxy-$N^2$-(1-oxo-octadecyl)-ornithine]-5-(3-hydroxyglutamine)-6-[3-hydroxyproline]-echinocandin B (formula Id)

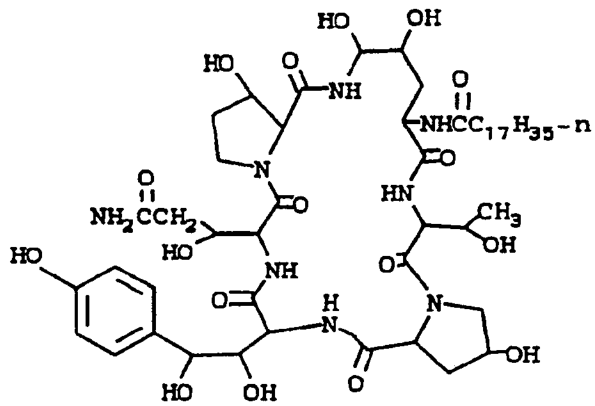

(Id)

is prepared by (1) intimately admixing with cooling stearic acid, pentafluorophenol and dicyclohexylcarbodiimide in ethyl acetate to obtain pentachlorophenyl stearate and (2) adding the pentachlorophenyl ester thus obtained to a solution of Compound A in dimethylformamide to obtain the compound of formula (Id) having a molecular weight of 1092.

EXAMPLE V

1-[4,5-Dihydroxy-$N^2$-(1-oxo-dodecyl)ornithine]-5-(3-hydroxyglutamine)-6-[3-hydroxy-proline]echinocandin B (Ie)

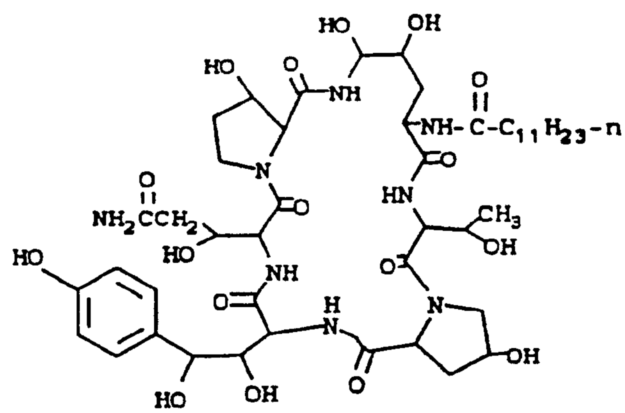

(Ie)

A. Preparation of pentafluorophenyl laurate

To a suspension of 1.001 grams of lauric acid in 10 milliliters of ethyl acetate was added 1.012 grams (5.5 mmol) of pentaflurophenol, followed by 1.134 grams (5.5 mmol) of dicyclohexylcarbodiimide and the mixture stirred at room temperature for four hours to obtain pentafluorophenyl laurate and dicyclohexyl-urea by-product.

The product was filtered the filter cake washed with ethyl acetate, and the filtrate and wash concentrated to obtain the pentaflurorophenyl laurate as 2.02 grams of oil.

B. Preparation of Compound Ie

To a suspension of 0.301 gram of Compound A (of 52 percent purity obtained by enzymatic deacylation) in 3 mL of dry DMF was added 0.305 g of the pentafluorophenyl laurate prepared as above described and the resulting mixture stirred at room temperature for 18 hours. At the end of this time, the DMF was removed under reduced pressure to obtain a residue which was triturated with ether. The mixture was filtered and the solid washed with ether. The solid was then placed in about 2 milliliters of mobile phase 70:30 ((95:5 water/acetonitrile)/(95:5 acetonitrile/water)), filtered ("Anotec" 25 plus filter) and then chromatographed (column: 25 cm x 24 mm "Zorbax" C8, elution with mobile phase) and approximately 20 milliliter fractions collected with detection at 230 nM. The fractions were monitored by analytical HPLC (column 5 mm x 25 cm "Zorbax" C8) and eluted with the same solvent system at a flow rate of 1.5 mL/min with detection at 210 nM and a $R_t$ of 10.08 minutes. The fractions having the desired retention time were combined, concentrated to remove the acetonitrile and the lyophilized to obtain Compound Ie.
MS(FAB): 1015 (M+Li)
$^1$H NMR (δ):7.13 (d, 2H, J=8.6 Hz), 6.74 (d, 2H, J=8.6 Hz), 5.27 (d, 1H, J=2.7 Hz), 5.08 (d, 1H, J=4.1 Hz), 4.98 (d, 1H, J=3.5 Hz), 1.16 (d, 3H, J= 6.1 Hz), 0.90 (t, 3H, J= 6.6 Hz).

EXAMPLE VI

1-[4,5-Dihydroxy-N$^2$-(1-oxo-tetradecyl)ornithine]-5-(3-hydroxyglutamine)-6-[3-hydroxyproline]echinocandin B (If)

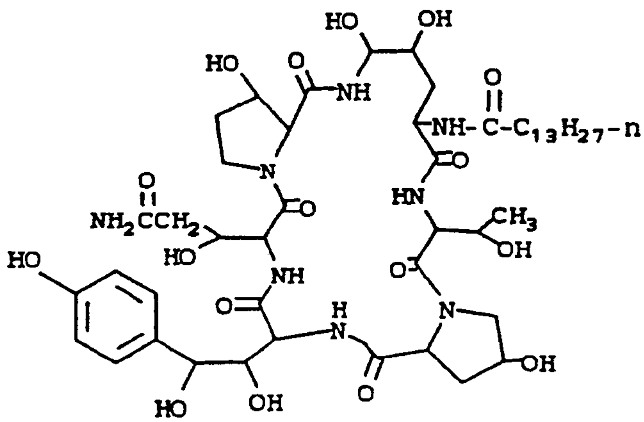

(If)

A. Preparation of pentafluorophenyl myristate

To a suspension of 1.141 grams of myristic acid in 10 milliliters of ethyl acetate was added 1.012 grams (5.5 mmol) of pentafluorophenol, then 1.34 grams (5.5 mmol) of dicyclohexyl carbodiimide and the mixture stirred at room temperature for four hours to obtain pentafluorophenyl myristate and dicyclohexylurea. The latter was removed by filtration, the filter cake washed with ethyl acetate and the wash and filtrate concentrated to obtain pentafluorophenyl myristate as 2.00 grams of white solid.

B. Preparation of Compound If

To a suspension of 0.300 gram of Compound A (52 percent purity) in 3 milliliters of dry DMF was added 0.305 gram of pentafluorophenyl myristate and an additional 1 millilter of DMF used to rinse the sides of the flask. The resulting mixture was stirred for about twenty-four hours. The DMF was then removed under reduced pressure and the residue remaining triturated with ether. The solid precipitate, recovered by filtration and air dried, amounted to 0.33 gram. The solid was taken up in a mixture of mobile phase 60:40 (95:5 water/acetonitrile)/(95:5 acetonitrile/water), filtered and chromatographed using the mobile phase composition for elution and at a flow rate of 10 mL/minute approximately 20 mL fractions were collected with detection at 230 nM. The fractions were monitored by analytical HPLC (Column 5 mm x 25 cm "Zorbax" C8 and eluted with the same solvent system at 45°C with a flow rate of 1.5 mL/min with detection at 210 nM and a $R_t$ of 7.84

minutes). The desired fractions were combined, concentrated to remove the acetonitrile and then lyophilized to obtain 0.06 gram of the desired myristic acid analog, Compound (If).
MS (FAB):1043 (M+Li)
$^1$H NMR (δ): 7.13 (d, 2H, J=8.6 Hz), 6.74 (d, 2H, J=8.6 Hz), 5.27 (d, 1H, J=2.7 Hz), 5.08 (d, 1H, J=4.1 Hz), 4.98 (d, 1H, J=3.2 Hz), 4.98 (d, 1H, J= 3.2 Hz), 1.16 (d, 3H, J=6.2 Hz), 0.09 (t, 3H, J= 6.6 Hz).

EXAMPLE VII

1-[4,5-Dihydroxy-N$^2$-(1-oxo-hexadecyl)ornithine]-5-(3-hydroxyglutamine)-6-[3-hydroxyproline]echinocanlin B (Ig)

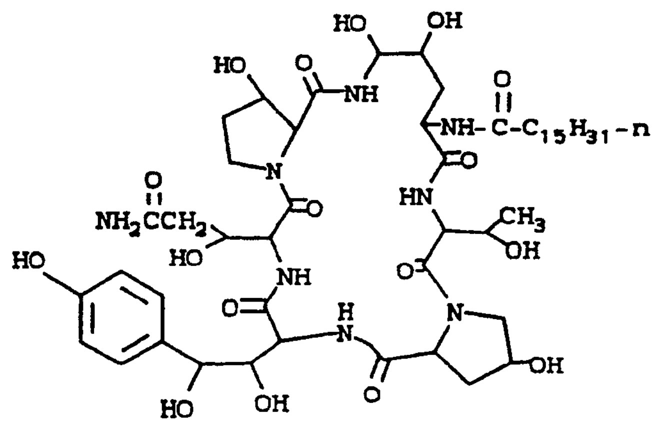
(Ig)

A. Preparation of pentafluorophenyl palmitate

In an operation carried out in a manner similar to that described in Example V and VI, a suspension of 1.282 grams (3 mmol) of palmitic acid in 10 milliliters of ethyl acetate, 1.012 grams (5.5 mmol) of pentafluroraphenol and 1.134 grams (5.5 mmol) of dicyclohexylcarbodiimide were stirred together for four hours to obtain pentafluorophenyl palmitate and dicyclohexylurea. The latter was removed by filtration, the filter cake washed with ethyl acetate and the ethyl acetate solution combined and concentrated under reduced pressure to obtain 2.20 grams of pentafluorophenyl palmitate as a white solid.

B. Preparation of Compound Ig

In a manner similar to that described in Example VI, 0.301 gram of Compound A (of 52 percent purity) in 3 milliliter of DMF, 0.305 grams of pentafluorophenyl palmitate (obtained as above described) and an additional 1 milliliter of DMF used to rinse flask were stirred at room temperature for 18 hours, and the mixture then concentrated to remove the DMF in vacuo and the residue triturated with ether. The mixture was filtered to obtain a gummy solid. The solid was taken up in the mobile phase of 65:35 95:5 water/acetonitrile to 95:5 acetonitrile/water then filtered ("Anotec" British Alcan Aluminum), and chromatographed ("Zorbax" C8 25 cm x 24 mm) and eluted with the mobile phase at a flow rate of 10 mL/minute and 20 milliliter fractions collected (detection at 230 nM). The columns were monitored by analytical HPLC (5 mm x 25 cm "Zorbax" C8) and eluted with the same solvent system as above described at 45°C with a flow rate of 1.5 ml/minute (detection at 210 nM). The desired fractions were combined, concentrated to remove the acetonitrile, then lyophylized to obtain 0.05 gram of Compound Ig.
MS (FAB): 1071 (M+Li)
$^1$H NMR (δ): 7.13 (d, 2H, J=8.6 Hz), 6.74 (d, 2H, J=8.6 Hz), 5.27 (d, 1H, J=2.5 Hz), 5.08 (d, 1H, J=4.2 Hz), 4.98 (d, 1H, J=3.3 Hz), 1.16 (d, 3H, J=6.2Hz), 0.90 (t, 3H, J= 6.6 Hz).

EXAMPLE VIII

1-[4,5-Dihydroxy-N$^2$-(2-benzofuroyl)ornithine]-5-(3-hydroxyglutamine)-6-[3-hydroxyproline]echinocandin B (Ih)

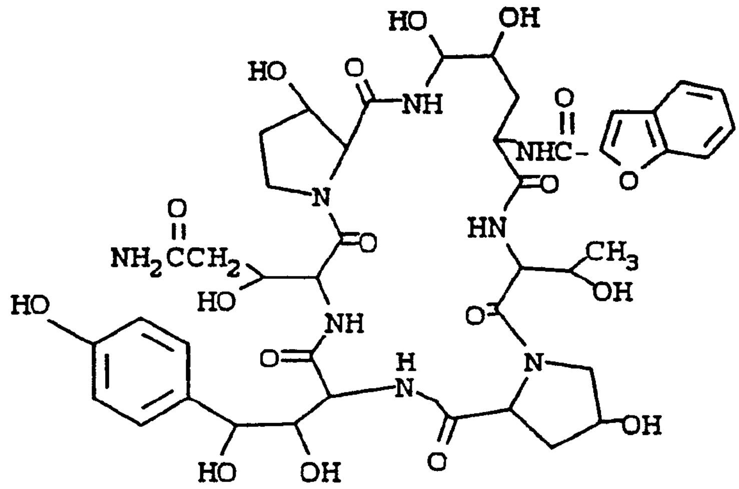

(Ih)

A. Preparation of pentafluorophenyl benzofuran-2-carboxylate

In a similar operation 2.26 gram of dicyclohexylcarbodiimide was added to a suspension of 2.01 g of pentafluorophenol and 1.62 g (10.0 mmol) of benzofuran-2-carboxylic acid in 20 milliliters of ethyl acetate and the mixture allowed to stir at room temperature for 2 hours to obtain the ester and dicyclohecylurea by-product. The latter was filtered, the filter cake washed with ethyl acetate and the filterate concentrated to obtain 3.67 grams of crude pentaflurophenyl benzofuran-2-carboxylate.

B. Preparation of Compound Ih

To a suspension of 0.301 gram of Compound A (52 percent purity) in 3 milliliters of dry DMF was added 0.31 gram of pentafluorophenyl benzofuran-2-carboxylate and the resulting solution allowed to stir at room temperature overnight. The DMF was then removed under reduced pressure and the residue triturated with ether, filtered and washed with ether to obtain 0.33 gram of crude Compound Ia.
MS (FAB): 977 (M+Li)

EXAMPLE IX

In reactions carried out in a manner similar to those described in the preceding examples, the following compounds wherein the molecular weight (M.W.) is that of the compound having R as designated are prepared:

## TABLE 1

| R | M.W. |
|---|---|
| $CH_3(CH_2)_5CH=CH(CH_2)_7-$ | 1062 |
| $CH_3(CH_2)_5CH=CH(CH_2)_9-$ | 1090 |
| $3-(n-C_8H_{17}O)C_6H_4$ - | 1058 |
| $4-(n-C_9H_{19}O)C_6H_4$ - | 1072 |
| $4-(n-C_{10}H_{21}O)C_6H_4$ - | 1086 |
| $4-(n-C_8H_{17})C_6H_4$ - | 1042 |
| $4-(n-C_9H_{17})C_6H_4$ - | 1056 |
| $4-(n-C_8H_{17}S)C_6H_4$ - | 1074 |
| $n-C_{10}H_{21}-$ | 994 |
| $i-C_6H_{13}-$ | 938 |
| $n-C_8H_{17}-$ | 966 |
| $4-(n-C_{10}H_{21}S)C_6H_4$ - | 1102 |
| $1-C_4H_9N$ - | 925 |
| $1-C_4H_9S$ - | 942 |
| $1-C_4H_9O$ - | 926 |
| 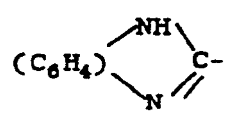 | 970 |
| 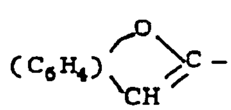 | 970 |
| 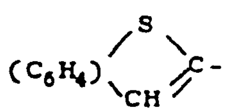 | 986 |

Preparation of the Compound A

Compound A may be prepared first by producing Compound B by the cultivation of Zalerion arboricola, isolating Compound B and thereafter subjecting Compound B to enzymatic deacylation by cultivating Compound B with Pseudomonas acidovorans or Pseudomonas diminuta, or one of the Actinoplanaceae organisms.

In producing Compound B, a frozen Culture of Zalerion arboricola ATCC 20868 or ATCC 20957 is inoculated into 54 milliliters of seed medium of the following composition: corn steep liquor, 5.0 g; tomato paste 40.0 g; oat flour 10.0 g; glucose 10.0 g; trace element mixture, 10.0 ml; all in 1000 liters of distilled water at pH 6.8. The trace element mixture contains per liter: $FeSO_4{\bullet}7H_2O$, 1 g; $MnSO_4{\bullet}4H_2O$, 1 g; $CuCl_2{\bullet}2H_2O$, 25 mg; $CaCl_2$, 100 mg; $H_3BO_3$, 56 mg; $(NH_4)_6Mo_7O_{24}{\bullet}4H_2O$, 19 mg; and $ZnSO_4{\bullet}7H_2O$, 200 mg.

The seed flasks are incubated from 3 to 6 days at 25°C with agitation. About 2 ml of resulting culture growth is inoculated into a production medium and incubated at 24° to 28°C for 3 to 30 days with or without agitation. The production medium may be liquid or solid. Representative of one preferred liquid production medium is of the following composition per liter: D-mannitol, 44 grams; $KH_2PO_4$, 2 grams; glycine, 2 grams; peptonized milk,

15 grams; lactic acid, 2 grams; trace element mixture (composition same as above), 10 ml; soybean oil, 10 g (pre-sterilization pH of 7.0). Representative of one preferred solid production medium is of the following composition per 250 ml flask: millet, 15 g and base liquid 15 ml. The base liquid is of the following composition per liter: ardamine PH (yeast autolysate, Yeast Products Inc., Clifton, NJ) 33.0 g; sodium tartrate, 6.6 g; $FeSO_4 \bullet 7H_2O$, 0.66 g; monosodium glutamate, 6.6 µg; and corn oil, 6.6 ml.

Other methods for the production of Compound B is described in the aforementioned copending application Serial No. 374,416, corresponding to EP-A-0405997.

The cultural and morphological characteristics of Zalerion arboricola ATCC 20868 or ATCC 20957 are as follows:

Colonies on potato-dextrose agar (difco) at 20°C slow-growing, attaining a diamter of. 8-12 mm in one week. Mature colonies (3-4 weeks on potato-dextrose agar effuse, with submerged and aerial hyphae, surface hairy, lanose, or funiculose, dull to moderately shiny, forming raised, densely compact colonies, with a substromatic texture due to dense conidia formation. Colony color pale olive-brown, olive, olive-brown, finally olive-black, Isabella Color, Sayal Brown, Tawny-olive, Saccardo's Umber, Sepia, Brownish Olive, Raw Umber, Dark Olive, Olivaceous Black (capitalized color names from R. Ridgway. 1912. Color Standards and Nomenclautre, Washington, D.C.). Same colors in colony reverse. Odor, exudates, and soluble pigments absent.

Hyphae,(in 3% KOH) pale yellow-brown to olive-brown, septate, branched, often with irregular lateral or terminal lobes, 1-3 um wide, thin- to slightly thick-walled, with walls smooth to lsightly incrusted or verrucose. Aerial hyphae often adhering together in fascicles. Setae arid hyphopodia absent.

Conidiogenous cells monoblastic, scattered to dense, integrated, terminal and intercalary, arising directly from undifferentiated hyphae, at right to slightly acute angles. Conidia originating as irregular chains, filaments, or coils, later developing as compact, irregular masses of 6-25 cells, Individual conidial cells, 3-6 um in diameter, globose, subglobose, or slightly irregular to loved, smooth to finely verruculose, yellow-brown to olive brown.

The Z. arboricola ATCC 20957 may be obtained by treating a spore suspension of Z. arboricola ATCC 20868 in 0.3 M tris(hydroxymethyl)aminomethane (TRIS) buffer pH=7 with N-nitroso-N-methylurethane, plating the treated suspension on potato dextrose agar and incubating to develop colonies, thereafter isolating the colonies, transferring the separate colonies to slants of potato dextrose agar and incubating for 10 to 14 days at 25°C to obtain cultures of mutants of Z. arboricola, one of which is ATCC 20957 as more fully described in Serial No. 492,024, corresponding to EP-A-0405997.

Compound B is then isolated from the fermentation mixture by extracting from the production medium with alcohol, preferably methanol, then partitioning the desired compound into a water-immiscible oxygenated organic solvent such as ethyl acetate, vaporizing the solvent and purifying the residue or concentrate by one or more chromatographic separations as more fully described in Serial No. 374,416, Serial No. 492,025, Serial No. 492,024 and Serial No. 492,026, corresponding to EP-A-0405997.

Compound A may then be produced by subjecting Compound B, obtained as above described, to the action of a deacylating enzyme.

The deacylating enzyme is first produced by inoculating a loopful of Pseudomonas acidovorans ATCC 53942 into 50 milliliters of Luria Bertani medium of the following composition: per liter Bacto-Trypton, 10 g; Bacto-Yeast Extract, 5 g and sodium chloride 10 g and solidified with 2 percent agar and incubating for 24 hours with shaking to obtain a seed culture. Cells for deacylation are then grown by diluting a 50 milliliter portion of seed culture 1:500 into fresh Luria-Bertani medium and incubating at 25°C with shaking for 16 hours. Cells are then harvested by centrifugation, washed by resuspending in 1 percent sodium chloride and centrifuged and then resuspended in potassium phosphate buffer at pH 6.5.

A solution of Compound B in dimethyl sulfoxide is then added to a stirred suspension of the cells of P. acidovorans thus obtained and the mixture maintained for 18 hours to obtain Compound A which is recovered in the supernatant by centrifugation.

Compound A may be isolated by adsorbing the supernatant on HP-20 resin with water, eluting with methanol and concentrating the eluates as more fully described in the aforementioned concurrently filed copending application Serial No. 492,001 (Attorney Docket No 17996).

The cultural and morphological characteristic of P. acidovorans ATCC 53942 are as follows: Gram-negative aerobic rod, approximately 0.8-1.0 µm x 3.0-4.0 µm. Growth occurs on trypticase soy agar at 25-37°C. Colonies are opaque and convex with an entire margin and glistening surface. Colonies have a butyrous texture. No pigments are observed. Growth on MacConkey agar is also observed.

The biochemical characteristics of this strain are as follows: oxidase positive, gelatin is hydrolyzed, nitrate reduced to nitrite. Growth occurs by assimilation of the following carbon sources in the presence of ammonium sulfate: D-gluconate, caprate, adipate, and malate, D-mannitol, and phenylacetate.

## Claims

1. A compound having the formula

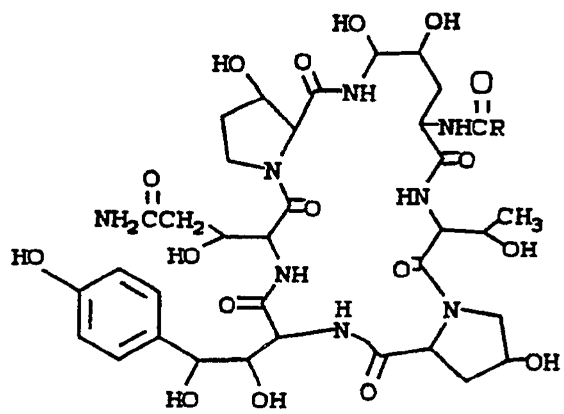

(I)

wherein R is
   (a) a straight or branched chain alkyl from 5 to 23 carbon atoms,
   (b) a straight or branched chain alkenyl from 5 to 23 carbon atoms,
   (c) phenyl and substituted phenyl wherein the substituent is $C_1$ to $C_{10}$ alkyl, $C_1$ to $C_{10}$ alkoxy or $C_1$ to $C_{10}$ thioalkoxy; or
   (d) heteroaryl selected from the group consisting of pyrryl, thiophenyl, furyl, indolyl, benzothiophenyl, benzofuryl, imidazolyl, benzimidazolyl, and pyridinyl.

2. An antimicrobial composition which comprises an antimicrobial amount of a compound of Claim 1 in admixture with a biologically inert carrier.

3. An antimicrobial composition which comprises a composition of Claim 2 in which the carrier is a pharmaceutically acceptable carrier.

4. A method for inhibiting fungal growth in consumer goods, plants and plant parts, plant products, wood and lumber, pulps and paper, and the like, comprising applying to the area where fungal growth is to be controlled an antifungally effective amount of the compound of Claim 1.

5. The use of a compound as claimed in Claim 1 for the manufacture of a medicament for the treatment of fungal infections.

6. The use according to Claim 5 in which the medicament is adapted for the treatment of mycotic infections.

7. The use of a compound as claimed in Claim 1 for the manufacture of a medicament for controlling Pneumocystis carinii infections in mammals.

European Patent Office

# EUROPEAN SEARCH REPORT

Application Number

EP 91 30 2070

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 031 662 (ELY LILLY & CO.,) | | C 07 K 7/56<br>A 61 K 37/02 |
| | | | TECHNICAL FIELDS SEARCHED (Int. Cl.5) |
| | | | C 07 K<br>A 61 K |

The present search report has been drawn up for all claims

| Place of search | Date of completion of the search | Examiner |
|---|---|---|
| THE HAGUE | 17-05-1991 | DEFFNER C-A.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)